# EUROPEAN PATENT APPLICATION

(11) **EP 2 253 306 A1**
(43) Date of publication of application: **24.11.2010**
(21) Application number: 09394011.2
(22) Date of filing: 18.05.2009
(51) Int. Cl.: A61K 9/00, A61K 9/20

(54) **Orodispersible dosage forms containing solid drug dispersions**

(71) Applicant: Royal College of Surgeons in Ireland, Dublin 2 (IE)
(72) Inventor: The designation of the inventor has not yet been filed
(74) Representative: Purdy, Hugh Barry

(57) **Abstract**

A solid crystalline drug dispersion in particulate form comprises drug crystals dispersed in a matrix formed of a disintegrant, wherein the drug crystals generally have a median diameter D50% of not greater than 20µ, the drug is a hydrophobic drug, and substantially all of the drug in the solid dispersion is in a crystalline form. The particles of solid dispersion have a median diameter D_{50%} of between 1 and 50µ. The disintegrant is a superdisintegrant selected from the group consisting of: a crosspovidone or derivatives thereof; crosscarmellose sodium or derivatives thereof; sodium starch glycollate or derivatives thereof; and calcium silicate. A method of producing a solid crystalline drug dispersion is also disclosed and comprises the steps of providing a crystalline drug in which the drug crystals have a median diameter D50% of not generally more than 20µ, preparing a dispersion or suspension of drug crystals in a disintegrant solution or suspension, and spray drying the dispersion or suspension to provide a particulate composition comprising particles of solid crystalline drug dispersion. An oral dose form comprising a solid crystalline drug dispersion of the invention is also described..

## Description

### Technical Field

The invention also relates to particulate solid drug dispersions, and oral dosage forms comprising particulate solid drug dispersions.

### Background to the Invention

Hydrophobic drugs generally have poor bioavailability due to their poor solubility. This problem is exacerbated when the drug is formulated as an as an immediate release tablet including fast disintegrating tablets (also known as fast melt-type tablets), which require fast dispersibility in the aqueous environment of the gastrointestinal tract or oral cavity . Various attempts to overcome the problem of the poor solubility and bioavailability of hydrophobic drugs have been described: for example, International Patent Application WO03/063831 describes immediate release dosage forms in which the drug is processed as a solid drug dispersion, which is then formulated with a disintegrant and a porosogen to form an orodispersible tablet. In the process described in this patent, the drug is solubilised in a concentration-enhancing polymer to form an amorphous dispersion of drug, and the dispersion is spray dried to form a particulate solid drug dispersion in which the drug is present in a predominantly amorphous form. It is well known that the amorphous form of a hydrophobic drug has greater dissolution characteristics than a crystalline form of the drug. Thus, the drug present in the solid dispersions of WO03/063831 would have good dissolution characteristics. However, molecules in an amorphous, high energy, form are also known to be less stable that those in a crystalline, low energy, form, and therefore the drug present in the solid dispersions of WO03/063831 are prone to reconvert to the low energy, crystalline form. This change can occur over time and can be accelerated in response to a number of different stimuli, including temperature, moisture, and physical processing. Thus, while the solid dispersions described in WO03/063831 initially provide good dissolution characteristics, the instability of the amorphous form of the drug leads to the reformation of drug crystals and an accompanying loss of solubility and bioavailability.

It is an object of the invention to overcome at least one of the above-referenced problems.

### Statements of Invention

### A. Solid Drug Dispersions

In a first aspect, the invention provides a solid crystalline drug dispersion in particulate form and comprising drug crystals dispersed in a matrix formed of a disintegrant. The drug crystals generally have a median diameter D50% of not greater than 100µ, typically 50µ, suitably 40µ, preferably 30µ, and more preferably 25µ. Ideally, the drug crystals have a median diameter D50% of not greater than 20µ.

Surprisingly, the Applicant has discovered that the formulation of solid drug dispersions using a disintegrant as a carrier provides excellent rheological properties while helping maintain the crystalline nature of the drug. Thus, the crystals of drug, which by virtue of their size have increased surface area, dissolution and hence bioavailability, are stably incorporated into the disintegrant matrix without any prominent change in polymorphic form. Further, as the crystals are homogenously dispersed through a matrix formed of a disintegrant, the particles of the solid dispersion break up quickly and easily in an aqueous environment, releasing the small crystals of drug quickly and in a controlled manner.

The term "drug" as used herein should be taken to mean a compound that exists in a crystalline form and which has therapeutic or prophylactic properties. Both hydrophilic and hydrophobic compounds are envisaged. In a preferred embodiment of the invention, the drug is a low-solubility drug. Typically, the low-solubility drug has an aqueous solubility at pH 7 of less than 0.01mg/ml, or 0.001mg/ml. Suitably, the drug is a hydrophobic drug, having a Clog P value of at least 3, typically at least 4, and preferably at least 5. Clog P is the base 10 logarithm of the ratio of the drug solubility in octanol to the drug solubility in water. Examples of hydrophobic drugs include the cholesterol-lowering drugs, including the statins simvastatin and atorvastatin, nonsteroidal anti inflammatory agents such as indomethacin, diclofenac, meloxicam and carprofen. Other examples of hydrophobic drugs include antihypertensives, anxiolytic agents, anticlotting agents, anticonvulsants, blood glucose lowering agents, decongestants, antihistamines, antitussives, antineoplastics, beta blockers, anti-inflammatory agents, antipsychotic agents, cognitive enhancers, anti-atherosclerotic agents, antiobesity agents, autoimmune disorder agents, anti-impotence agents, and antibacterial and antifungal agents.

Suitably, at least 50% (w/w) of the drug in the solid dispersion is in a crystalline form. Preferably, at least 60%, 70%, 80%, or 90% (w/w) of the drug in the solid dispersion is in a crystalline form. Ideally, substantially all of the drug in the solid dispersion is in a crystalline form.

Suitably, the crystalline drug has a median diameter D_{50%} of not greater than 20µ ,10µ, 5µ or 1µ. In a preferred embodiment of the invention, the crystalline drug is in the form of nanocrystals. Typically, the nanocrystals have a D_{50%} of not greater than 1000nm, 800nm, 500nm, 300nm or 100nm. Methods for producing drug crystals in micron (micronization) and nano- sizes will be well known to those skilled in the art. In particular, the production of nanocrystals is described in US6316029, US5145684, US5298262, US5302410, US5318767, and numerous other documents described in Paragraph 0017 of US2003/0215502.

The term "solid crystalline drug dispersion" should be understood as meaning solid particles produced by spray drying, although other suitable drying process such as solvent evaporation can be used, and having the drug in a predominantly crystalline form dispersed throughout a carrier matrix. Typically, the particles of solid dispersion have a median diameter D_{50%} of 1 to 1000µ, suitably less than 250µ, preferably less than 100µ, and ideally between 1 and 50µ. In one embodiment, the solid particles are selected from the group consisting of: granules; microparticles; and nanoparticles.

The carrier for the drug comprises a disintegrant. The disintegrant forms a solid matrix and the drug is dispersed in the matrix. The disintegrant aids in the break up of the solid dispersion when in an aqueous environment. Surprisingly, the Applicant has discovered that the use of a disintegrant, and especially a superdisintegrant, as a carrier for the drug helps prevent the drug changing into an amorphous forms during processing. Typical disintegrants include hydroxypropyl cellulose (HPC), especially low substituted hydroxypropyl cellulose (L-HPC) as defined in USP, sodium starch glycollate, carboxymethyl cellulose, crosscarmelose sodium, crosspovidone, and starch, and derivatives thereof. Ideally, the disintegrant is a superdisintegrant, examples of which will be well known to those skilled in the art. In this specification, the term "superdisintegrant" means a disintegrant that has a Eq. Moisture content at 25C/90%RHn of over 50%. Ideally, the superdisintegrant is selected from the group consisting of: crosspovidone (i.e KollidonCL, CLSF, CLM, Polyplasdone XL) or derivatives thereof; crosscarmellose sodium (i.e. Ac-Di-sol, Primellose, Vivasol) or derivatives thereof; sodium starch glycollate (i.e. an EXPLOTAB, such as primojel, DMV, V17 or CLV) or derivatives thereof; calcium silicate; other polysaccharides such as soy polysaacharide, EMCOSOY, cellulose derivatives including HPC, alginic acid (SATIALGINE H8). Ideally, the superdisintegrant is a crosspovidone.

The ratio of drug to disintegrant in the solid dispersion is from 100:1 1 to 1:100 (w/w), typically from 20:1 to 1:20 (w/w), and preferaby from 10:1 1 to 1:10 (w/w). Suitably, the ratio of drug to disintegrant is from 1:10 to 1:1, preferably from 1:3 to 1:10, and ideally from 1:3 to 1:5 (all w/w).

The solid dispersion of the invention has good flow properties. Typically, the solid dispersion has a Carr's Index of less than 25, 20, 15, 10. Ideally, the solid dispersion has a Carr's index of less than 15.

The invention also relates to a method of producing a solid crystalline drug dispersion comprising the steps of providing a crystalline drug in which the drug crystals have a median diameter D50% of preferably not more than 20µ, preparing a dispersion or suspension of drug crystals in a disintegrant solution or suspension, and spray drying the dispersion or suspension to provide a particulate composition comprising particles of solid crystalline drug dispersion.

Ideally the dispersion or suspension of drug is mixed for at least 5, 10, 15, 20, 30 minutes prior to spray drying to allow a uniform suspension. This will depend on product volume and mixing equipment used

Ideally, the disintegrant is a superdisintegrant. Examples of suitable disintegrants and superdisintegrants are provided above.

Typically, the disintegrant is formulated as an aqueous solution or suspension. Suitably, the disintegrant solution or suspension has a disintegrant concentration of from 1 to 25% (w/v), typically from 2.5 to 10% (w/v). Generally, the dispersion or suspension has a concentration of drug of from 1 to 30%, preferably from 1 to 10% w/v, more preferably between 2 to 5% w/v.

The process generally involves first preparing a solution or suspension of the disintegrant, and then subsequently adding the drug to the solution or suspension and mixing to disperse or suspend the drug crystals in the disintegrant solution.

The term "spray drying" should be taken to mean conventional spray drying (in which a feed solution is sprayed into droplets into a stream of hot gas or air which solidifies the droplets to provide particles, or spray chilling in which the droplets are hardened by passing through a chilled gas. Typically, the nozzle employed in the spray drier has a diameter of between 0.7 and 4 mm.

Suitably, the flow rate of the dispersion or suspension stream to the nozzle is up to 25ml/min depending on the viscosity of the stream and the pump setting. The droplets formed by the nozzle are dried as they leave the nozzle and pass through the heated gas. In a spray drying process, the gas is hot air or a heated inert gas such as nitrogen, typically having an inlet temperature of between 80°C and 220°C (preferably between 90°C and 110°C, and ideally about 100°, when heated nitrogen is used). Suitably, the heated nitrogen has an outlet temperature of between 40°C and 70°C.

When heated air is used, the inlet temperature typically has a range 120-220°C and the outlet temperature typically a range of between 60°C and 160°C.

When spray chilling is employed, the dispersion or suspension stream may comprise lipids including phospholipids, waxes, surfactants such as polyethylene glycols, or low melting point polymers, all of which preferably having a melting point of 75°C or less.

The invention also relates to a solid crystalline drug dispersion obtainable by the method of the invention.

### B. Oral Dose Forms

The invention also relates to an oral dose form comprising a solid crystalline drug dispersion of the invention. The term "oral dose form" should be understood to mean any form of drug that is intended to be taken orally, and would include, for example, directly compressed tablets, capsules, powders for reconstitution as oral suspensions, wafers and other solid oral dose forms. The oral dose form may also include one or more pharmaceutically acceptable excipients, for example, sweeteners, flavoring agents, osmotic agents, effervescent agents, disintegrants, direct compression fillers, lubricants, glidants and the like.

In one preferred embodiment of the invention, the oral dose form is a tablet, typically an orodispersible tablet, comprising a solid crystalline drug dispersion according to the invention. Typically, the tablet comprises a filler, solid crystalline drug dispersion, and optionally one or more of a lubricant, a flavoring agent, a sweetening agent, an osmotic agent, and a flow enhancer. Typically, the tablet is directly compressed. In one embodiment of the invention, the filler is a direct compression (DC) filler, for example a pre-processed granulated sugar-based filler. Preferably, the only disintegrant (or superdisintegrant) in the tablet is that which forms part of the solid dispersion. Typically, the tablet does not include any flow enhancer.

In this specification, the term "orodispersible tablet" should be taken to mean that the tablet has a disintegration time of 60 seconds or less. Typically, the tablets have a hardness of at least 30N, and a disintegration time of less than 60 seconds. Ideally, the tablets have a disintegration time of less than 40 seconds and a hardness of at least 60N.

Thus, the invention relates to a directly compressed tablet comprising:
- 1 to 60% (w/w) of a solid crystalline drug dispersion of the invention; and
- 1 to90% (w/w) of a DC filler.

Generally, the drug comprises from 1 to 50%, suitably from 5 to 25%, of the tablet (w/w). The drug may be a high dose active, or a low dose active. When, the drug is a high dose active, it is generally included in the tablet in an amount of at least 50mg, 75mg, 100mg, 125mg and 150mg.

The invention also relates to a method of producing a tablet, the method comprising the steps of directly compressing a mixture of components at a compression force of at least 4 kN to form the tablet wherein the mixture of components comprises a solid crystalline drug dispersion of the invention and a filler, typically a DC filler.

The invention also relates to a method of producing an orodispersible tablet, the method comprising the steps of directly compressing a mixture of components to form the tablet, wherein the mixture of components comprises a solid crystalline drug dispersion of the invention and a DC filler. The mixture of components optionally also includes a disintegrant, a flavouring agent, a lubricant, and a flow enhancer.

Other methods of producing tablets such as granulation can also be used.

Where the methods of the invention involve the tablets being formed in a direct compression process, a tablet press is generally employed. In a preferred embodiment, the direct compression process employs substantially flat faced toolings. Thus, the thickness of the formed tablet will not vary considerably from the centre to the edges (unlike tablets produced using bi-convex toolings which are thicker in the middle that at the edges). Typically, the flat faced toolings have a uniform depth, which will not vary in thickness between the centre and edge by more that +/- 5%, preferably 4%, preferably 3%, more preferably 2%, and ideally by more than 1%. Ideally, the tablets have a bevelled edge and will typically disintegrate within 60 seconds.

The term "DC filler" should be taken to mean a sugar, a polyol, or a sugar alcohol. In one preferred embodiment, the DC base is a DC sugar alcohol. Examples of suitable DC sugar alcohols are Mannitol 100, Mannitol 200, Mannitol 300 and Mannitol 400. Preferably, the DC base is Mannitol 200 or 100. Other types of sugar-based direct compression bases include lactose fast flow, lactose DC, Sorbitol Instant, sucrose, dextrose, xylitol, and maltitol. Suitably, the sugar-based direct compression base is included in an amount of from 1 to 90%, typically from 20 to 80%, and ideally from 30 to 70y% (w/w).

The term "DC filler" may also include a microcrystalline cellulose (MCC) direct compression base. Typically, the MCC base is Avicel. In one preferred embodiment, the MCC base is a silicified MCC base. These bases comprise an intimate physical mixture of colloidal silicon dioxide with microcrystalline cellulose (see for example US Patent 5,585,115). Suitable examples of silicified MCC are ProSolv 50 and ProSolv 90 (Penwest), having an average particle size of 50µ and 90µ, respectively. In a preferred embodiment, the silicified MCC is ProSolv 90. Suitably, the MCC base is included in an amount of from 1 to 90%, typically from 20 to 80y%, and ideally from 30 to 70% (w/w). In one embodiment, the DC filler comprises MCC and no DC sugar.

Ideally, the tablets have a hardness of at least 40N, and a disintegration time of less than 15 minutes preferably less than 5 minutes and more preferably less than 1 minute and ideally less than 90, 60, 40, 30, 20, 15 or 10 seconds.

In one embodiment, the tablet has a friability of less than 1%, as per USP, method, and typically less than 0.6%, and ideally less than 0.2 %.

The lubricant is typically selected from the group comprising: magnesium stearate; stearic acid, polyethylene glycol, polyoxyethylene- polyoxypropylene block copolymer (poloxamer). Suitably, the lubricant comprises between 0.1% and 5.0%, preferably between 0.2% and 1.0%, of the tablet (w/w).

In another embodiment, the lubricant, instead of or in addition to being included in the tablet formulation, is coated on to the faces of the tabletting punches and dies.

The flow enhancing agent may consist of talc or colloidal silicon dioxide, at from 0.1% to 3.0%, and preferably from 0.1% and 0.5%, of the tablet (w/w).

The flavouring agent may be, for example, synthetic oils, natural oils, or extracts from plants or other suitable synthetic or naturally derived flavors), typically at a level ranging from 0.5 to 5 % of the tablet (w/w).

The mixture of components may also include a surfactant or wetting agent (such as sodium lauryl sulphate, Tweens, Spans), typically at a level of from 0.1 to 3% of the tablet (w/w).

In a particularly preferred embodiment, the tablet has diameter in the range of 5-20mm, preferably in the range of 10-15mm and more preferably 15mm. Typically, the tablet has a diameter of at least 10mm, at least 11mm, at least 12mm, at least 13mm, and at least 14mm. Preferably, the tablet has a thickness of between 1 and 6 mm, preferably between 1.5-3.5 mm.

In a preferred embodiment of the invention, the compression force employed in the direct compression process is from 4kN to 20kN, typically from 8 kN to 15kN.

In a preferred embodiment of the invention, the tablet is substantially flat-faced. Ideally, the tablet has a bevelled edge. Suitably, the tablet is generally circular, although other shapes of tablets are envisaged such as oval, rectangular, triangular and square.

The invention also relates to a pharmaceutical capsule containing a mixture of components which includes a solid crystalline drug dispersion of the invention in combination with one or more pharmaceutical excipients.

The invention also relates to a fast disintegrating and dispersing powder containing a mixture of components which includes a solid crystalline drug dispersion of the invention in combination with one or more pharmaceutical excipients including an effervescent excipients. Typically, the fast disintegrating and dispersing powder is suitable for filling into oral sachets as individual dose or for filling into a multidose bottle as an oral powder for reconstitution as an oral suspension.

### Brief Description of the Figures

Figure 1 . Scanning electron micrographs of (a) pure simvastatin and solid dispersions of simvastatin and (b) Povidone K30 (c) Povidone VA64 and (d) Crospovidone, KollidonCLSF.
Figure 2: DSC thermograms of (a) povidone K-30, povidone:simvastatin (b) 1:3 Solid Dispersion (c) 1:3 Physical Mix, (d) Solid Dispersion 9:1 and (e) 1:9 Physical Mix.
Figure 3: X-Ray diffraction analyses of samples: (a) background measurement, (b) pure simvastatin, (c) simvastatin: Povidone K-30 1:3 solid dispersion (d) simvastatin:
   Povidone K-VA 64 1:3 solid dispersions, physical mixtures of simvastatin, at 1:3 ratio, with (e) povidone K-30, (f) povidone K-VA 64.
Figure 4: DSC thermograms of (a) Kollidon VA64, (b) simvastatin: Kollidon VA64 (1:3) solid dispersion and (c) simvastatin: Kollidon VA64 (1:3) Physical Mix.
Figure 5: DSC thermograms of (a) Kollidon CLSF, simvastatin: Kollidon CLSF (b) 1:1 solid dispersion, (c) 1:2 solid dispersion (d) 1:2 Physical Mix (e) 1:9 solid dispersion (f) 9:1 solid dispersion and (g) 1:9 Physical Mix.
Figure 6: DSC thermograms of (a) Explotab, (b) simvastatin: Explotab 1:1 solid dispersion and (c) simvastatin: Explotab 1:2 Physical Mix.
Figure 7: DSC thermograms for (a) Calcium silicate, (b) simvastatin:Ca Silicate 1:1 solid dispersion and (c) simvastatin: Ca Silicate 1:2 Physical Mix.

### Detailed Description of the Invention

### Experimental

The process of the invention employs a conventional lab scale spray drier fitted with a conventional nozzle system which allows solid dispersions of a drug in a suitable carrier to be prepared. The drug and carrier is formulated as a suspension or dispersion (known as the feed dispersion) in an aqueous medium preferably and is sprayed from the nozzle into a stream of hot air. The dispersion droplets dry on contact with the hot air resulting in the formation of drug/carrier particles. The resulting solid dispersions of drugs demonstrate ease and speed of redispersibility upon contact with an aqueous medium such as water, buffers or simulated gastrointestinal fluids, to give the original drug particles without altering the polymorphic nature of the drug. It is known that spray drying of solutions of drugs with/without a suitable polymeric carrier often results in changes of the drug to a metastable polymorph or an amorphous form. This process is commonly utilised in the formulation of poorly soluble drug compounds in order to increase the solubility and dissolution rate of the drug compounds. Such practice however often demonstrate poor stability due to reconversion of the drug to its stable polymorph giving rise to a decrease in solubility, dissolution rate of the drug and decreased bioavailability.

It is the intention of this invention to formulate poorly soluble drug compounds as an easily redispersible matrix or dispersion without introducing any changes to the polymorphic form of the drug and which on contact with an aqueous fluid will redisperse to give the original drug particles hence maintaining desirable particle size for optimal solubility and dissolution characteristics and preserving stability of the drug. The original drug particles may be as small as required with average diameter below 20 microns, preferably below 10 microns and more preferably below 1 micron.

Using this process, the poorly soluble drug simvastatin was formulated as aqueous dispersions with a range of pharmaceutical carriers which have disintegrant properties. The dispersions were spray dried to give fast dispersible solid particles. The carriers used included crospovidone crosslinked polyvinylpyrrolidone (Kollidon CLSF), calcium silicate and sodium starch glycollate. The water soluble polymers, Polyvinylpyrrolidone (Kollidon 30) and polyvinyl povidone-VA64, vinylpyrrolidone-vinyl acetate copolymer (Kollidon VA64) were also studied as carriers which have no disintegrant properties. The spray dried solid dispersions prepared were analysed for polymorphism of the drug using differential scanning calorimetry (DSC) using a TA Q100 instruments), X ray diffraction (XRD, Bruker D8 Discover) and Fourier transform infrared (FT-IR) using a Bruker Tensor 27 FT-IR spectrometer. The spray dried formulations were also analysed for particle size by laser sizer (Malvern Mastersizer 2000) using the Scirocco™ dry powder attachment, morphology by scanning electron microscopy (SEM) using a Tescan Mira Variable Pressure Field Emission Scanning Electron Microscope (VPFESEM). The drug content of the dispersions was measured using high Performance Liquid Chromatography (HPLC) as per the method described in the United States Pharmacopeoia, USP 30 and National Formulary, NF 25, 2007.

### Procedure for spray drying of fast dispersible solid dispersions.

The feed dispersion was pumped through the single nozzle using the integral Bucchi peristaltic pump of the spray drier. The solutions were sprayed at a rate of 1-4 ml per minute, equivalent to a Bucchi integral pump setting of 16-4%, into the drying chamber at an air inlet temperature of 90°C. The outlet temperature monitored throughout the drying process was similar for all formulations, ranging from 31°C to 48°C.

The spray drying was carried out using a Buchi B-290 mini spray dryer in an open mode cycle where compressed air is used for the drying of aqueous based formulations. Alternatively, the spray dryer can also be used in the closed cycle when the B-295 inert loop is switched on to provide drying in an inert atmosphere i.e., in absence of air. The closed cycle mode is used when the formulations are prepared using inflammable solvents including ethanol, acetone, dicholoromethane, toluene, ethylacetate. The solutions were spray dried in an atmosphere of heated air. Other spray drying parameters such as air aspiration rate and spray flow rate were held constant at 100% and 35mm, respectively.

In this invention, the one liquid feed was continuously fed through the single nozzle and atomized using compressed air. The droplets were then dried in an atmosphere of heated air using an inlet temperature of 100°c and the outlet temperature monitored during the process was in the range of 31°C-48°C. The air flow rate used was 350Nl/hour and the aspirator setting used was 40m³/hour. Generally, during spray drying, besides atomizing a continuous liquid feed, a rotary atomizer or ultrasonic atomiser can also be employed.

In another embodiment, spray drying can be carried out using a concentric double feed nozzle where the drug or drug and carrier dispersion is sprayed through the internal nozzle and carrier dispersions with and without additional excipients such as flavours, tablet fillers and binders and additional disintegrant are sprayed through the outer nozzle to produce a tablet blend of enhanced homogeneity, palatability, flow properties and disintegration. This dispersion provides a ready dispersible tablet blend suitable for direct compression into tablets including fast dissolving tablets, for filling into hard gelatin capsules or for filling into powder sachets and bottles for use as oral powders and reconstituted oral suspensions.

Spray drying techniques are well known to those skilled in the art and are described in the 'Spray Drying Handbook' by K. Masters, John Wiley & Sons, New York, 1984 and in the Buchi spray drying training papers, Buchi Labortechnik AG 1997, 1998.

Details of the formulation and spray drying parameters are given in Examples 1-5 below.

### Example 1 (comparative)

A solution of povidone (Kollidon 30, PVP) was prepared by dissolving 2.814g of povidone carrier in 50mls of deionised (DI) water in a Duran bottle using a magnetic stirrer (Yellowline, MSC basic C, IKA works, USA). The mixture was stirred at 500 - 600 rpm for approximately 20-30mins. After dissolution of povidone, (PVP) in water, 0.938g of simvastatin was weighed and transferred to this solution and further stirred at the same speed for approximately the same time. A dispersion of simvastatin in the aqueous PVP solution containing 7.5% w/v of total solid content, and drug to carrier ratio of 1:3 was obtained.

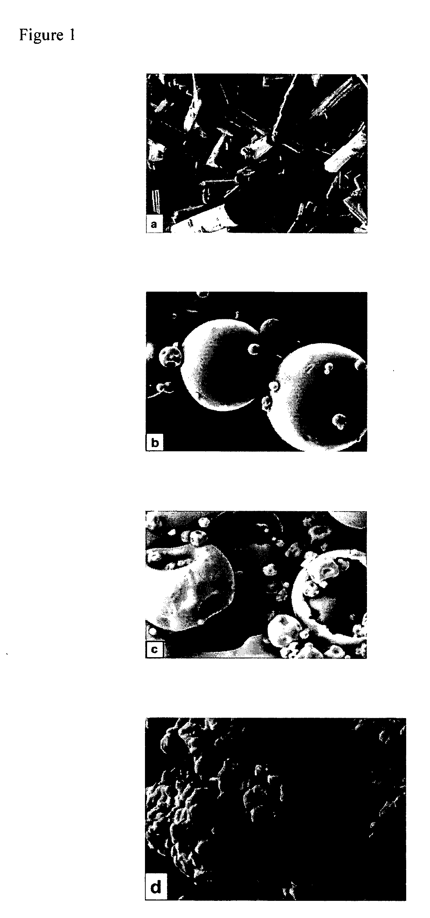

The feed dispersion prepared was spray dried according to the procedure described above. The integral Bucchi pump was used at a setting of 16. The PVP and simvastatin solid dispersions obtained was characterized for size by a Malvern Mastersizer Model 2000 fitted with the dry dispersion Scirocco™ attachment for dry powder analysis. The spray dried particles had a median diameter (D_{50%}) of 6.87 +/-0.39 microns. Scanning electron microscopy image of the solid dispersions showed smooth spherical particles were formed (Figure 1a) giving a free flowing powder with a measured Carr's index of 0. Assay of simvastatin content carried out by high performance liquid chromatography as per USP 2007 method, showed a simvastatin content of 15.39% +/- 0.75% w/w. Simvastatin pure drug material has a median diameter (D_{50%}) of 7.39 +/-0.03 microns and has a reduced flowability showing a Carr's index of 17.5 ± 2.57, a reflection of the elongated crystal morphology of the material (Fig. 1a). The formulation of simvastatin with PVP showed improved morphology reflecting in excellent flowability of the drug: povidone solid dispersion

Example 1 was repeated for the weight ratios of simvastatin:PVP of 9:1. Using an aqueous feed dispersion of 7.5% w/w of total solid (simvastatin and povidone) content. The solid dispersion obtained was charcaterised and the data obtained is shown in Table 1.

**Table 1. Characteristics of spray dried dispersions of simvastatin**

| Carrier | simvastatin: carrier weight ratio | Batch # | D_{50%} Microns | Theoretical simvastatin content (%w/w) | Assayed simvastatin content (%w/w) |
|---|---|---|---|---|---|
| K-30 | 1:3 | SIM39 | 6.87 ± 0.39 | 25 | 15.29 ± 0.75 |
| | 9:1 | SIM52 | 7.71 ± 0.04 | 90 | 85.54 ± 1.73 |
| K-VA64 | 1:3 | SIM40 | 5.92 ± 0.22 | 25 | 12.35 ± 2.42 |
| CaS | 1:1 | SIM41 | 5.76 ± 0.42 | 50 | 47.89 ± 3.11 |
| SSG (Explotab) | 1:1 | SIM46 | 7.45 ± 0.19 | 50 | 71.59 ± 4.79 |
| K-CLSF | 1:1 | SIM36 | 12.09 ± 0.55 | 50 | 45.36 ± 0.11 |
| | 1:2 | SIM35 | 14.15 ± 0.05 | 33.33 | 32.19 ± 1.08 |
| | 1:9 | SIM53 | 11.60 ± 0.05 | 10 | 10.31 ± 0.79 |
| | 9:1 | SIM54 | 8.14 ± 0.06 | 90 | 90.71 ± 3.31 |

At the higher drug: povidone ratio of 9: 1, the median particle size was 7.71 ± 0.04 microns similar to the median particle size of the pure simvastatin. The measured Carr's index of the 9:1 drug: povidone dispersion was 13.05 similar to the Carr's index of the pure drug material. The simvastatin content of the dispersion was 85.54 ± 1.7% w/w giving a drug loading efficiency of 95.04%.

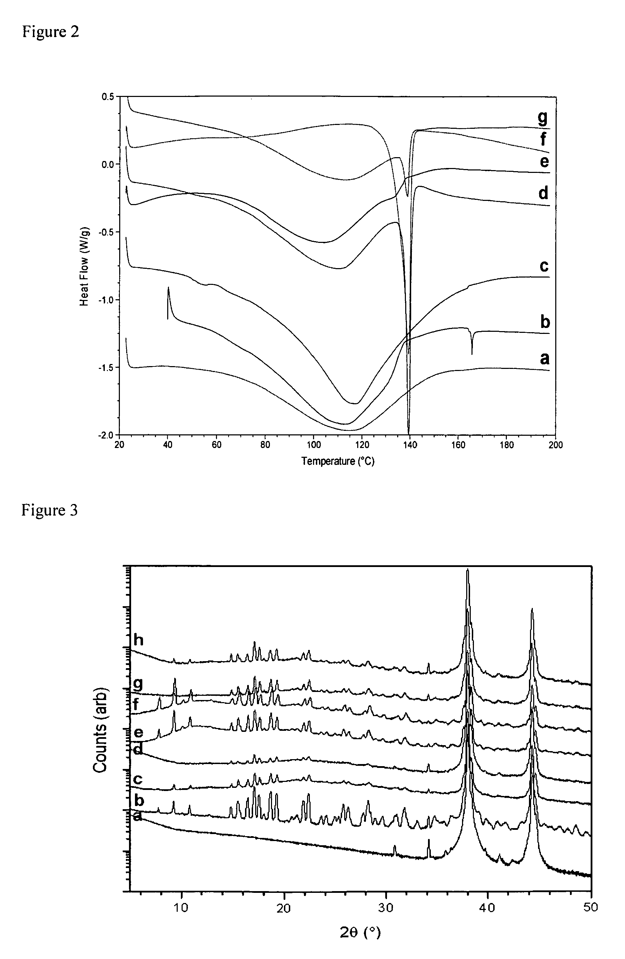

Differential scanning calorimetry (DSC) of the solid dispersions was carried out and is shown in Figure 2. Pure simvastatin showed a sharp endothermic event at 140.01 °C indicative of the crystalline nature of simvastatin. Povidone K-30 showed a broad endotherm with peak temperature of peak temperature at 116.54°C. At 1:3 ratio of drug to povidone, a glass transition temperature at ∼55°C was observed suggesting the conversion of simvastatin to an amorphous form. No difference in peak temperature of the endotherm related to povidone K-30 was observed. At the higher ratio of 9:1 drug to carrier, simvastatin showed a sharp endotherm indicating the presence of crystalline simvastatin. A reduced crystallinity of simvastatin was observed on XRD analysis for the 1:3 ratio of drug : povidone compared with the physical mix of the 1:3 ratio of simvastatin: povidone, shown in figure 3c and e respectively.

### Example 2 (comparative)

Example 1 was repeated using polyvinylpyrrolidone/vinyl acetatecopolymer, Kollidon VA64 (PVP VA64) as the carrier. A solution of Kollidon VA64 was prepared by dissolving 2.814g of the povidone VA64 carrier in 50mls of DI water in a Duran bottle using a magnetic stirrer on a magnetic plate and stirrer (Yellowline, MSC basic C, IKA works, USA). The mixture was stirred at 500 - 600 rpm for approximately 20-30mins. After dissolution of povidone VA64 in water, 0.938g of simvastatin was weighed and transferred to this solution and further stirred at the same speed for approximately the same time to give a dispersion containing 7.5%w/v of total solid content, and drug to carrier ratio of 1:3.

Using the process and equipment as described above, the feed dispersion containing simvastatin and povidone VA64 was spray dried to give solid dispersions. Laser diffraction analysis of the spray dried particles showed the particles had a median, (D_{50%}), diameter of 5.92 ± 0.22 microns (Table 1). Electron microscopy showed the product to be discrete microparticulates with puckered surface, Fig. 2c, giving rise to very good flow properties, Carr's index of 7.13. The assayed simvastatin content of the solid dispersion was 12.35 ± 2.42 % w/w.

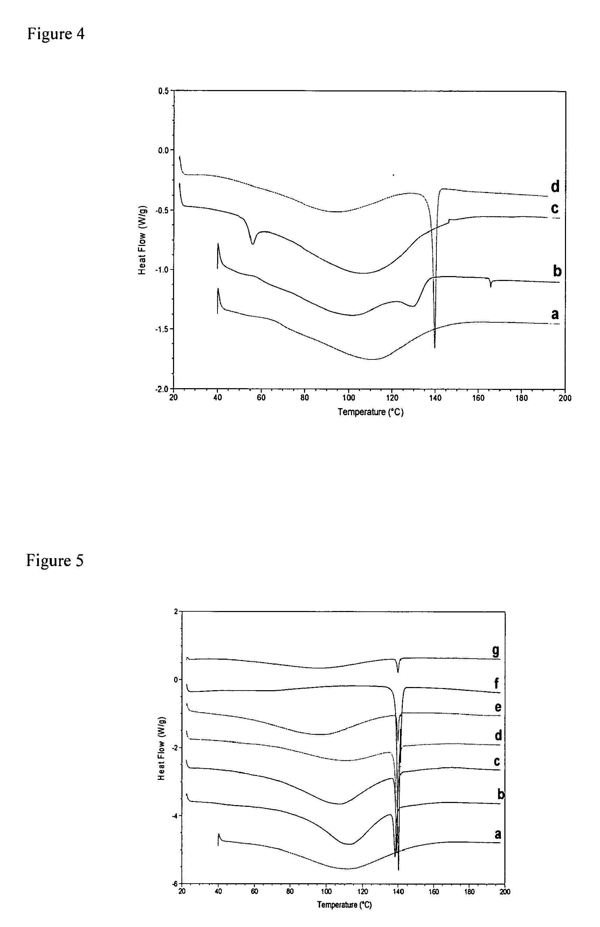

DSC analysis (Figure 4) conducted on the solid dispersion showed the presence of amorphous simvastatin as demonstrated by the glass transition temperature at ∼55°C. Physical mix of simvastatin and the povidone VA64 showed the sharp endotherm at a peak temperature of 139.83°C for the presence of crystalline simvastatin. The broad endotherm at 91.80°C is characteristic of the carrier K-VA64. The reduced crystallinity of simvastatin is confirmed by XRD as shown in Figures 3 d and f

Both povidone K30 and povidone VA64 are water soluble and are known to enhance the solubility of poorly soluble drugs. Without being bound by theory, it is possible that simvastatin was partly solubilised by each of these 2 carriers resulting in the formation of amorphous simvastatin dispersed in the povidone carriers and this accounts for the lower particle size and morphology of the spray dried dispersions observed. This is a surprising finding. As povidone, or povidone VA64 are water soluble, these carriers can be used at certain ratios of carrier to drug to prepare a fast dissolving matrix to release a fine dispersion of the drug in predominantly a crystalline state.

### Example 3

Crospovidone is crosslinked povidone which is used as a superdisntegrant. Crospovidone is water insoluble and hence stays in suspension with simvastatin unlike dispersions prepared in Examples 1 & 2.

A dispersion of corospovidone (Kollidon CLSF) was prepared by transferring the 2.5g of the carrier in 50mls of deionised water in a Duran bottle using a magnetic stirrer on a magnetic plate and stirrer (Yellowline, MSC basic C, IKA works, USA). It was stirred at 500 - 600 rpm for approximately 20-30mins. After dispersion in water, 1.25g of simvastatin was weighed and transferred to this solution and further stirred at the same speed for approximately the same time to give a dispersion containing 7.5%w/v of total solid content at a drug to carrier ratio of 1:2 was prepared.

Using the process and equipment as described in Example 1, the feed dispersion containing simvastatin and crospovidone Kollidon CLSF was spray dried to give solid dispersions. Laser diffraction analysis of the spray dried particles showed the particles had a median, (D_{50%}), diameter of 14.15 ± 0.05 microns (Table 1). Electron microscopy showed the product to be irregular material, Fig. 2d. The dispersion showed excellent flow properties with a Carr's index of 5.79. The simvastatin content of the solid dispersion showed a simvastatin content of 32.19 ± 1.08 % w/w. DSC studies carried out on this solid dispersion (Figure 5) shows a sharp endothermic event at about 140°C, corresponding to simvastatin in its original crystalline form. A broader endothermic event at 110-115°C relates to the crospovidone melting event.

### Example 4

Example 3 was repeated for simvastatin: crospovidone (Kollidon CLSF) weight ratios of 1:1, 1:9 and 9:1. The aqueous feed dispersion used had a total solids content of 7.5% w/w. The characterization data of these dispersions are shown in Table 1. Laser diffraction analysis of the spray dried particles showed the particles had a median, (D_{50%}), diameter of 8.14 +0.06 to 12.09 ± 0.55 microns (Table 1). The dispersion showed excellent flow properties with a Carr's index in the range of 4.71 and 10.33 for the 1:1 and 9:1 ratios respectively. At the lowest ratio of 1:9 simvastatin to crospovidone CLSF, the Carr's index measured was 16.68. The simvastatin content of the solid dispersion showed a simvastatin content in the range of 90.71 to 103.1% w/w of theoretical simvastatin content. DSC studies carried out on the solid dispersions (Figure 5) showed a sharp endothermic event with peak melting point in the range of 139°C-140°C corresponding to simvastatin in its original crystalline form and a broader endotherm related to the presence of the crospovidone, Kollidon CLSF.

### Example 5

Example 3 was repeated for simvastatin: Explotab weight ratios of 1:1 . A dispersion of Explotab in deionised water was prepared by transferring the 1.675g of the carrier in 50mls of deionised water in a Duran bottle using a magnetic stirrer on a magnetic plate and stirrer (Yellowline, MSC basic C, IKA works, USA). It was stirred at 500 - 600 rpm for approximately 20-30mins. After dispersion in water, 1.675g of simvastatin was weighed and transferred to this solution and further stirred at the same speed for approximately the same time to give a dispersion containing 7.5%w/v of total solid content at a drug to carrier ratio of 1:1 was prepared. The dispersion was spray dried using the process described in Example 1. The characterisation data of this dispersion are shown in Table 1. Laser diffraction analysis of the spray dried particles showed the particles had a median, (D_{50%}), diameter of 7.45 ± 0.19_microns (Table 1). The dispersion showed good flow properties with a Carr's index in the range of 19.1. The simvastatin content of the solid dispersion showed a higher simvastatin content at 71.59 ± 4.79 % w/w, at 143% of theoretical simvastatin content. This higher drug content is attributed to the larger particle size of the Explotab compared with simvastatin resulting in sedimentation of part of the Explotab during spray drying. This can be improved by for example continuous mixing of the feed dispersion during processing and/or adding a suspending agent or viscosity enhancing agent. Other methods are known to those skilled in the art.

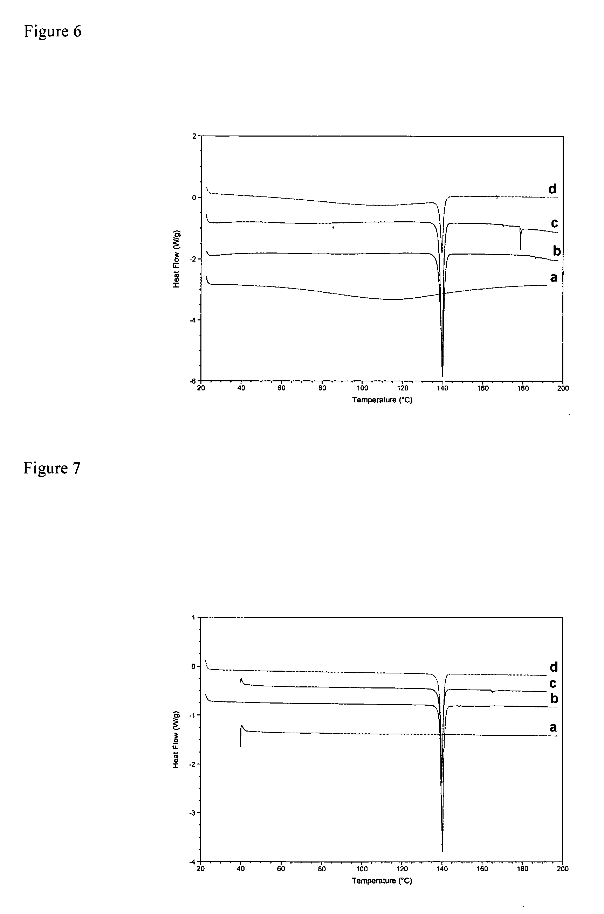

DSC analysis of the solid dispersions (Figure 6) showed a sharp endothermic event with peak melting point at around 140°C corresponding to simvastatin in its original crystalline form and a broader endotherm at ∼115-120°C related to the presence of the Explotab.

### Example 6

Example 3 was repeated for simvastatin: Calcium silicate ratios of 1:1. A dispersion of Calcium silicate in deionised water was prepared by transferring the 1.675g of the carrier in 50mls of deionised water in a Duran bottle using a magnetic stirrer on a magnetic plate and stirrer (Yellowline, MSC basic C, IKA works, USA). It was stirred at 500 - 600 rpm for approximately 20-30mins. After dispersion in water, 1.675g of simvastatin was weighed and transferred to this solution and further stirred at the same speed for approximately the same time to give a dispersion containing 7.5%w/v of total solid content at a drug to carrier ratio of 1:1 was prepared. The dispersion was spray dried using the process described in Example 1. The characterisation data of this dispersion are shown in Table 1. Laser diffraction analysis of the spray dried particles showed the particles had a median, (D_{50%}), diameter of 5.76 ±0.42 microns (Table 1). The dispersion showed excellent flow properties with a Carr's index in the range of 10.0. The simvastatin content of the solid dispersion showed a simvastatin content of 47.89 ± 3.11 % w/w, at 95.8% of theoretical simvastatin content.

DSC analysis of the solid dispersions (Figure 7) showed a sharp endothermic event with peak melting point at around 140°C corresponding to simvastatin in its original crystalline form.

The examples 3 to 6 above show that when the crystalline drug is formulated with a disintegrant and processed to form solid drug dispersion particles, the drug predominantly retains its crystal morphology. In comparison, and referring to Examples 1 and 2 in which the carrier is a water soluble polymer which does not have disintegrant properties, the DSC studies show conversion of the drug to an amorphous state. at the ratio of 1:3 drug to polymer,

### Example 7

Formulation of fast disintegrating tablets containing 20mg simvastatin as spray dried simvastatin dispersion

Fast disintegrating tablets containing 20 mg of simvastatin as the spray dried solid crystalline dispersion were compressed using 13 mm flat faced beveled edge tooling at a weight of 300 mg per tablet. The solid dispersion used were at 1:1 and 1:2 ratios of simvastatin:crospovidone CLSF to give batches 2008/039 & 2008/040 respectively. Details of the composition of the formulations used are given in table 2. The solid dispersion was blended with the Mannitol 200 and the flavours first using bag blending. The Magnesium stearate was added to the blend and was mixed for 5 minutes (using bag blending) prior to tabletting. Tablets were compressed using an 8-tooling automated tablet press (Piccola, Riva,UK) The compression force used was 10KN and tablets were produced at both the low compression speed of 7 rpm and at the highest compression speed of 49 rpm.

**Table 2. Composition of fast disintegrating tablets containing simvastatin as solid crystalline dispersion in Crospovidone CLSF matrix.**

| Active/Excipients | Batch# 2008/039(%) | Batch # 2008/040 (%) |
|---|---|---|
| Simvastatin:Crospovidone (KollidonCLSF® ) solid crystalline dispersion | 13.4 | 20 |
| Mannitol 200 (Emprove^{®} Parteck^{®} M 200) | 84.5 | 77.9 |
| Mg Stearate | 0.5 | 0.5 |
| Crospovidone | - | - |
| Raspberry flavour | 0.8 | 0.8 |
| Mint flavour | 0.8 | 0.8 |

The tablets produced were characterised for weight uniformity using a Sartorious CP225D weighing scale. The content of simvastatin was assayed using HPLC analysis, The tablet thickness was measured using Workzone digital Callipers. Tablet hardness and friability were determined using test methods as described in the BP 2008, methods 2.9.8 & 2.9.7 respectively. The disintegration time of the tablets were measured by a modification of the method (method 2.9.1) described for conventional tablets in BP 2008. Instead of performing the test on n=6 tablets at one time, n=6 tablets were individually tested and the disintegration time recorded. The data obtained for tablets produced at 49 rpm is shown in table 3.

Tablet weights showed little variability and were 296.23 ± 8.46 mg for batch 2008/039 and 294.90 ± 7.86 mg for batch 2008/040. The average disintegration times observed were below 20 seconds at 15.17 ± 3.49 seconds for batch 2008/039 and batch xxx respectively. Tablet hardness of 35.48 ± 9.83N were recorded respectively for batch 2008/039 and 2008/040. Both batches had low friability at 0.24% to 0.27%, much lower than 1% limit for conventional tablets and below the 2% USP limit for fast disintegrating tablets.

**Table 3. Characteristics of fast dissolving tablets formulated using simvastatin as solid crystalline dispersion.**

| Batch number | Weight (mg) | Hardness (N) | Thickness (mm) | DT (sec) | Friability (% wt loss) | Assayed simvastatin content (%) |
|---|---|---|---|---|---|---|
| 2008/039 | 296.23 ± 8.46 | 35.48 ± 9.83 | 2.25 ± 0.03 | 15.17 ± 3.49 | 0.24 | 91.78 ± 5.13 |
| 2008/040 | 294.90 ± 7.86 | 35.48 ± 9.83 | 2.38 ± 0.02 | 18.50± 4.55 | 0.27 | 89.21 ± 3.98 |

The invention is not limited to the embodiments hereinbefore described which may be varied in construction and detail without departing from the spirit of the invention.

## Claims

1. A solid crystalline drug dispersion in particulate form and comprising drug crystals dispersed in a matrix formed of a disintegrant, wherein the drug crystals have a median diameter D50% of not greater than 20µ.

2. A solid crystalline drug dispersion as claimed in Claim 1 in which the drug is a hydrophobic drug.

3. A solid crystalline drug dispersion as claimed in any preceding Claim in which at least 90% (w/w) of the drug in the solid dispersion is in a crystalline form.

4. A solid crystalline drug dispersion as claimed in Claim 3 in which substantially all of the drug in the solid dispersion is in a crystalline form.

5. A solid crystalline drug dispersion as claimed in any preceding Claim in which the crystalline drug has a median diameter D_{50%} of not greater than 10µ.

6. A solid crystalline drug dispersion as claimed in Claim 5 in which the crystalline drug is in the form of nanocrystals.

7. A solid crystalline drug dispersion as claimed in any preceding Claim in which the particles of solid dispersion have a median diameter D_{50%} of between 1 and 50µ.

8. A solid crystalline drug dispersion as claimed in any preceding Claim in which the disintegrant is a superdisintegrant.

9. A solid crystalline drug dispersion as claimed in Claim 8 in which the superdisintegrant is selected from the group consisting of: a crosspovidone or derivatives thereof; crosscarmellose sodium or derivatives thereof; sodium starch glycollate or derivatives thereof; and calcium silicate.

10. A solid crystalline drug dispersion as claimed in any preceding Claim in which in which the ratio of drug to disintegrant in the solid dispersion is from 1:10 to 1:1 (w/w).

11. A solid crystalline drug dispersion as claimed in any preceding Claim in which the solid dispersion has a Carr's index of less than 15.

12. A method of producing a solid crystalline drug dispersion comprising the steps of providing a crystalline drug in which the drug crystals have a median diameter D50% of not more than 20µ, preparing a dispersion or suspension of drug crystals in a disintegrant solution or suspension, and spray drying the dispersion or suspension to provide a particulate composition comprising particles of solid crystalline drug dispersion.

13. An oral dose form comprising: a solid crystalline drug dispersion of any of Claims 1 to 12; or a solid crystalline drug dispersion obtainable by a method of Claim 12.

14. An oral dose form as claimed in Claim 13 provided in a form selected form the group consisting of: tablets; capsules; powders for reconstitution as oral suspensions and wafers.

15. An oral dose form as claimed in Claim 13 or 14 provided in the form of a directly compressed tablet.
